# EUROPÄISCHE PATENTSCHRIFT

(11) **EP 3 823 587 B1**
(45) Veröffentlichungstag und Bekanntmachung des Hinweises auf die Patenterteilung: **23.04.2025**
(21) Anmeldenummer: 19742171.2
(22) Anmeldetag: 16.07.2019
(51) Int. Cl.: A61K 9/00, A61K 9/08, A61M 1/16, A61P 7/08, A61K 33/10

(54) **VERFAHREN ZUR STABILISIERUNG EINER DIALYSELÖSUNG**
METHOD FOR STABILIZING A DIALYSIS SOLUTION
PROCÉDÉ DE STABILISATION D'UNE SOLUTION DE DIALYSE

(30) Priorität: 17.07.2018 DE 102018117264
(43) Veröffentlichungstag der Anmeldung: 26.05.2021
(73) Patentinhaber: Fresenius Medical Care Deutschland GmbH, 61352 Bad Homburg (DE)
(72) Erfinder: LAUBROCK, Andreas, 61273 Wehrheim (DE); HUPPERT, Jochen, 66132 Saarbrücken (DE); MATHIS, Pascal, 66793 Saarwellingen (DE); BERLICH, Robert, 66606 St. Wendel (DE)
(74) Vertreter: Herrmann, Uwe
(86) Internationale Anmeldenummer: PCT/EP2019/069070
(87) Internationale Veröffentlichungsnummer: WO 2020/016206

(56) Entgegenhaltungen:
- WO-A1-2016/202462
- PIERIDES A M ET AL: "The need and use of a phosphate-enriched dialysate during regular hemodialysis", TRANSACTIONS OF AMERICAN SOCIETY FOR ARTIFICIAL INTERNAL ORGANS, ASAIO INC. BOCA RATON, FLORIDA, US, vol. 23, 1 January 1977 (1977-01-01), pages 376 - 384, XP009160456, DOI: 10.1097/00002480-197700230-00097
- TODD S ING ET AL: "REVIEW ARTICLES Phosphorus-Enriched Hemodialysates: Formulations and Clinical Use", 9 May 2003 (2003-05-09), XP055182550, Retrieved from the Internet <URL:http://onlinelibrary.wiley.com/store/10.1046/j.1492-7535.2003.00023.x/asset/j.1492-7535.2003.00023.x.pdf?v=1&t=i8fwft1a&s=3df20eb20088d0784077c5e8e5627c9a8ef517af> [retrieved on 20150413]

## Beschreibung

Die Erfindung betrifft ein Verfahren zur nachträglichen Stabilisierung einer Dialyselösung gegenüber der Ausfällung von Calciumcarbonat.

Aus der WO 2016/041634 A1 ist bekannt, dass geringe Mengen an ortho-Phosphat im millimolaren Konzentrationsbereich die Ausfällung von schwerlöslichem Calciumcarbonat aus Dialyselösungen verhindern kann. Des Weiteren ist aus der WO 2016/202462 A1 bekannt, dass auch organische Phosphate wie zum Beispiel Glycerophosphat einen stabilisierenden Effekt ausüben können, ebenso Gemische aus ortho-Phosphaten und organischen Phosphaten. Die Stabilisierung erfolgt dabei im Stand der Technik durch initiale Zugabe einer definierten Phosphatmenge bei der Herstellung der Lösung.

Viele am Markt erhältliche Dialyselösungen enthalten keine Phosphate als Stabilisierungsagenzien und sind somit anfällig für Fällungsreaktionen.

Aufgabe der Erfindung ist es, ein Verfahren bereitzustellen, um die Anfälligkeit derartiger Lösungen für unerwünschte Fällungsreaktionen zu verringern.

Vor diesem Hintergrund betrifft die Erfindung ein Verfahren zur Stabilisierung einer Dialyselösung, die Calciumionen sowie Bicarbonationen enthält, wobei der Dialyselösung in zeitlichem Abstand zu deren Herstellung ein Phosphat in einer Menge von bis zu 0,4 mmol/I und/oder ein organischer Phosphatester in einer Menge von bis zu 1,25 mmol/I zugegeben wird, der zeitliche Abstand zur Herstellung mehr als 30 Minuten, mehr als 60 Minuten, mehr als 90 Minuten oder mehr als 105 Minuten beträgt und der pH-Wert der Lösung im Zeitpunkt der nachträglichen Zugabe größer als 7,2 ist.

Die Erfindung hat also die zeitversetzte Zugabe (Spiking) eines Phosphats bzw. eines organischen Phosphatesters zur Stabilisierung bestehender Dialyselösungen zum Gegenstand. Es wurde überraschend herausgefunden, dass nicht nur durch eine initiale, sondern auch durch eine nachträgliche Zugabe eines Phosphats oder organischen Phosphatesters noch ein stabilisierender Effekt gegen die Ausfällung von Calciumcarbonat erzielt werden kann. So inhibiert die nachträgliche Zugabe des Phosphats trotz der bereits vorhandenen Keime weitere Ausfällungen. Eine Auflösung bestehenden Niederschlags wurde nicht beobachtet.

Der Begriff der Dialyselösung ist weit zu verstehen und umfasst alle Lösungen, die während einer Dialysebehandlung zum Einsatz kommen können, also neben Lösungen für die Hämodialyse beispielsweise auch Substitutionslösungen oder Lösungen für die Peritonealdialyse. Besonders bevorzugt ist der Einsatz des erfindungsgemäßen Verfahrens aber an Lösungen für die kontinuierliche Nierenersatztherapie (CRRT), insbesondere Substitutionslösungen. Substitutionslösungen werden direkt ins Blut des Patienten infundiert und dürfen deshalb keine Partikel enthalten.

Der Begriff der Dialyselösung umfasst nach dem Verständnis der vorliegenden Offenbarung auch solche Dialyselösungen, die noch in Form zweier oder mehrerer getrennter Einzellösungen vorliegt, deren Vermischen zum Erhalt einer gebrauchsfertigen Dialyselösung führt. Solche Einzellösungen können beispielsweise in einem Doppelkammerbeutel vorliegen. So kann im Rahmen des erfindungsgemäßen Verfahrens vorgesehen sein, eine Einzellösung mit einem Phosphat bzw. organischem Phosphatester zu spiken. Dabei kann eine Zugabe zu einer Einzellösung bevorzugt sein, die Bicarbonationen enthält und/oder keine Calciumionen enthält.

Wenn in der vorliegenden Offenbarung von Phosphaten bzw. Phosphatestern die Rede ist, dann sind von diesem Begriff immer sowohl die vollständig protonierten und teilweise protonierten Säuren sowie die Salze umfasst. Besonders bevorzugt ist im Rahmen der Erfindung die Zugabe von Salzen, insbesondere Natriumsalzen der Phosphate bzw. Phosphatester.

**In** einer Ausführungsform ist vorgesehen, dass es sich bei dem Phosphat um *ortho-*Phosphat handelt und/oder dass es sich bei dem organischen Phosphatester um einen organischen Ester des ortho-Phosphats handelt.

**In** einer Ausführungsform ist vorgesehen, dass es sich bei dem organischen Ester des ortho-Phosphats um ein Glycerin-ortho-Phosphat handelt. Dieser Stoff ist bereits als Wirkstoff beispielsweise bei der parenteralen Ernährung etabliert und auch im Europäischen Arzneibuch monographiert (01/2009:1995). Dieses relativ kleine Molekül kann schnell unter Freisetzung von ortho-Phosphat verstoffwechselt werden. Das Glycerin-ortho-phosphat kann ein Glycerin-2-*ortho*-phosphat, ein Glycerin-3-*ortho-*phosphat oder eine Mischung daraus sein.

Gemäß der Erfindung ist vorgesehen, dass das Phosphat in einer Menge von bis zu 0,4 mmol/l zugegeben wird. Bevorzugt kann eine Zugabe von bis zu 0,375 mmol/l, bis zu 0,25 mmol/l oder bis zu 0,2 mmol/l sein. In einer Ausführungsform ist vorgesehen, dass das Phosphat in einer Menge von mindestens 0,05 mmol/l zugegeben wird. Derartige Phosphatkonzentrationen liegen unterhalb physiologischer Konzentrationswerte, sodass die medizinische Wirksamkeit der Dialyselösung nicht beeinflusst wird.

Gemäß der Erfindung ist vorgesehen, dass der organische Phosphatester in einer Menge von bis zu 1,25 mmol/l zugegeben wird. Bevorzugt kann eine Zugabe von bis zu 1,2 mmol/l sein. In einer Ausführungsform ist vorgesehen, dass der organische Phosphatester in einer Menge mindestens 0,8 mmol/l zugegeben wird. Bevorzugt kann eine Zugabe von mindestens 1,0 mmol/l sein. Eine PhosphatKonzentration von 0,8 bis 1,25 mmol/l und vorzugsweise von 1 bis 1,2 mmol/l entspricht einer Konzentration, die dazu verwendet werden kann, den Phosphathaushalt von Dialysepatienten zu regulieren und beispielsweise einer Hypophosphatämie vorzubeugen. So kann im Fall von phosphatfreien Dialyselösungen durch nachträgliche Zugabe organischer Phosphatester und insbesondere Glycerophosphat nachträglich eine physiologisch wünschenswerte Phosphatkonzentration unter gleichzeitiger Erhöhung der Stabilität gegenüber Ausfällungsreaktionen von Calciumcarbonaten eingestellt werden.

In einer Ausführungsform ist vorgesehen, dass der Lösung in zeitlichem Abstand zu deren Herstellung sowohl ein Phosphat als auch ein organischer Phosphatester zugegeben wird, wobei vorzugsweise vorgesehen ist, dass das Konzentrationsverhältnis zwischen dem Phosphat und dem organischen Phosphatester zwischen 0,3/0,7 und 0,9/0,1 liegt. Bei Zugabe von sowohl einem organischen Ester der Phosphorsäure als auch von ortho-Phosphat kann ein synergischer Effekt auftreten, der zu einer noch besseren Stabilisierung der Lösung gegenüber der Ausfällung von Calciumcarbonat führen kann.

In einer Ausführungsform ist vorgesehen, dass der zeitliche Abstand zur Herstellung mehr als 60 Minuten, mehr als 90 Minuten oder mehr als 105 Minuten beträgt. Selbst gealterte Lösungen mit einem bereits erhöhten pH-Wert können im Rahmen eines erfindungsgemäßen Verfahrens also nachträglich stabilisiert und damit sicherer gemacht werden.

In einer Ausführungsform ist vorgesehen, dass der zeitliche Abstand zur Herstellung mehr als eine Woche, mehr als ein Monat, mehr als sechs Monate oder mehr als ein Jahr beträgt. Selbst gealterte Lösungen mit einem bereits erhöhten pH-Wert können im Rahmen eines erfindungsgemäßen Verfahrens also nachträglich stabilisiert und damit sicherer gemacht werden.

Erfindungsgemäß ist vorgesehen, dass der pH-Wert der Lösung im Zeitpunkt der nachträglichen Zugabe größer als 7,2 ist. Bevorzugt sind Werte größer als 7,4 oder größer als 7,6. Der pH-Wert frisch hergestellter Dialyselösungen liegt typischerweise niedriger und kann beispielsweise zwischen etwa 7,0 und 7,6 liegen. Der pH-Wert der Lösung im Zeitpunkt der nachträglichen Zugabe sollte aber möglichst noch unter 8,0 liegen, da bei einem pH-Wert von 8,0 oftmals bereits in signifikantem Ausmaß Calciumcarbonat ausgefallen ist und somit die Zugabe nicht mehr effektiv wäre.

In einer Ausführungsform enthält die Dialyselösung neben den Calciumionen auch weitere Elektrolyte, vorzugsweise Natriumionen, Kaliumionen, Magnesiumionen und/oder Chloridionen. Auch Magnesium kann als schwerlösliches Carbonat ausfallen und auch diese Fällungsreaktion kann anhand des Phosphats bzw. Phosphatesters potentiell inhibiert werden.

In einer Ausführungsform enthält die Dialyselösung oder Substitutionslösung ferner wenigstens ein Osmotikum, beispielsweise ein Saccharid wie etwa Glukose oder ein Derivat davon.

Beispielsweise können in der Dialyselösung die genannten Lösungsbestandteile unabhängig voneinander in den in Tabelle 1 genannten Konzentrationen vorliegen.

**Tabelle 1**

| | |
|---|---|
| Calciumionen: | 1-2 mmol/l, beispielsweise 1,5 mmol/l |
| Magnesiumionen: | 0,2-0,8 mmol/l, beispielsweise 0,5 oder 0,75 mmol/l |
| Kaliumionen: | bis zu 8 und vorzugsweise bis zu 4 mmol/l |
| Natriumionen: | 120-160 mmol/l, beispielsweise 140 mmol/l |
| Bicarbonationen (inkl. Carbonat-Ionen und gelöstem CO₂): | 30-40 mmol/l, beispielsweise 35 mmol/l |
| Osmotikum: | 4-12 mmol/l, beispielsweise 5,6 mmol/l |
| Chloridionen: | 100-120 mmol/l, beispielsweise 109 mmol/l |

Weitere Einzelheiten und Vorteile der Erfindung ergeben sich aus den nachfolgend anhand der Figuren beschriebenen Versuchen und Ausführungsbeispielen. In den Figuren zeigen:
- Figur 1:: einen beispielhaften Verlauf des pH-Wertes einer Dialyselösung über deren Lebensdauer;
- Figur 2:: eine schematische Darstellung chemischer Vorgänge während der Herstellung und während des Lebenszyklus einer Dialyselösung;
- Figur 3:: Werte für pHₘₐₓ und t_{G} im Experiment des Beispiels 1;
- Figur 4:: Phototrodensignal für ausgewählte Datenpunkte im Experiment des Beispiels 1;
- Figur 5:: Werte für pHₘₐₓ und t_{G} im Experiment des Beispiels 2; und
- Figur 6:: Phototrodensignal für ausgewählte Datenpunkte im Experiment des Beispiels 2.

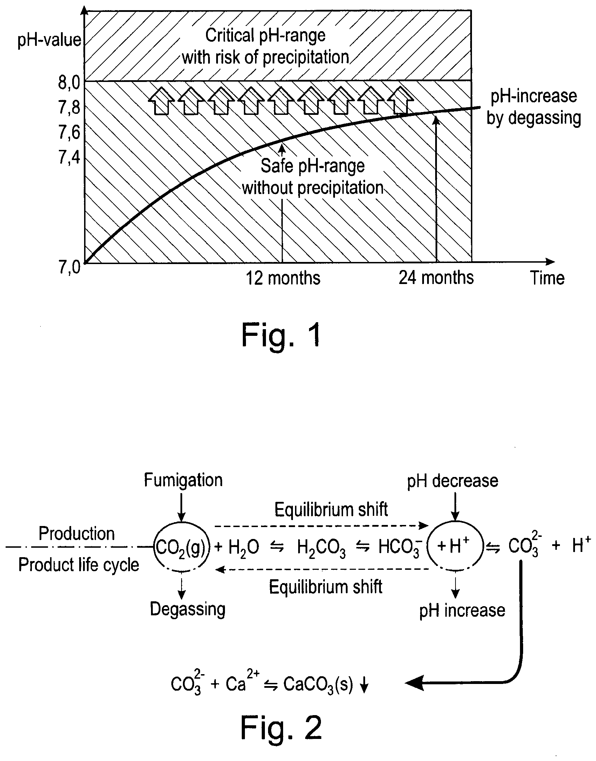

Durch die CO₂-Ausgasung aus einer bicarbonatgepufferten Dialyselösung wird der pH-Wert der Lösung erhöht, was im Falle calciumhaltiger Lösungen die Ausfällung von Calciumcarbonat begünstigt. Der pH-Wert, an dem eine maßgebliche Ausfällung von Calciumcarbonat einsetzt, ist ein Kriterium für die Stabilität der Lösung und wird nachfolgend als pHₘₐₓ bezeichnet. Je höher der Wert für pHₘₐₓ. Umso stabiler ist die Lösung. Der Zeitpunkt, an dem die maßgebliche Ausfällung von Calciumcarbonat einsetzt, wird nachfolgend in Anlehnung an den englischsprachigen Begriff *"time of germination"* als t_{G} bezeichnet. Ein beispielhafter Verlauf des pH-Wertes einer Dialyselösung über deren Lebensdauer ist in Figur 1 dargestellt, wobei der pHₘₐₓ in dieser Darstellung bei 8,0 angenommen wird und t_{G} in der gezeigten Zeitspanne noch nicht erreicht wird.

Der stabilisierende Effekt einer nachträglichen Zugabe von Phosphat bzw. einem Phosphatester zu bestehenden Dialyselösungen wurde anhand der Rapid-Degassing Methode im Karussell-Setup untersucht. Die Rapid-Degassing Methode in dieser Variante erlaubt eine beschleunigte Alterung von Dialyselösungen durch Ausgasen des CO₂ aus dem Bicarbonatpuffer. Das Ausgasen von CO₂, das normalerweise langsam während der Lebensdauer der Produkte auftritt, kann mit diesem Versuchsaufbau innerhalb weniger Stunden simuliert werden.

Die chemischen Vorgänge bei der Herstellung und dem Ausgasen der Lösung sind schematisch in Figur 2 dargestellt. So wird bei der Herstellung CO₂ in die Lösung eingebracht, um den pH-Wert der Lösung zu verringern. Während des Lebenszyklus der Lösung kehrt sich dieser Prozess durch Ausgasvorgänge nach und nach um, wodurch, bei resultierend höheren pH-Wert, ab einem bestimmten Punkt eine unerwünschte Ausfällung von Calciumcarbonat einsetzt.

### Beispiel 1:

Die Dialyselösung DUOSOL 4551 der Firma B.Braun wird in mehreren Experimenten mit und ohne Zugabe von ortho-Phosphat einer simulierten Alterung im Rahmen des oben beschriebenen Rapid-Degassing Verfahrens bei 40°C ausgesetzt. Die calciumhaltige Dialyselösung DUOSOL 4551 enthält keine Phosphate als Stabilisierungsagentien und ist daher anfällig für Ausfällungsreaktionen.

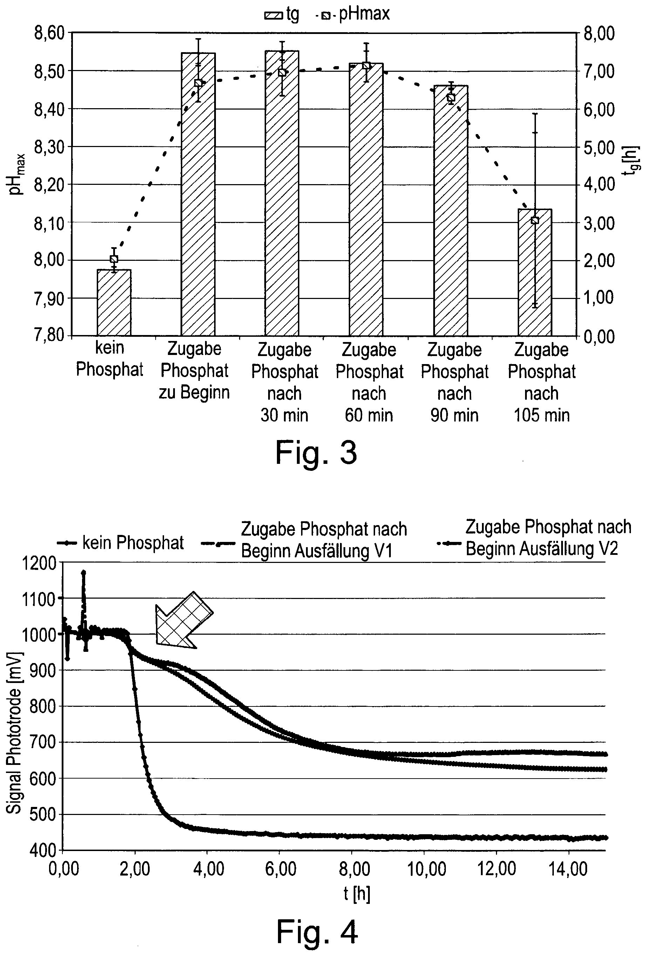

Zu mehreren Kolben enthaltend 250 ml DUOSOL 4551 werden nach einem bestimmten Zeitraum 250 µl einer Stammlösung mit 100 mmol/I ortho-Phosphat zugesetzt, was im Kolben zu einer Konzentration von 0,1 mmol/I an ortho-Phosphat führt. Für jeden Datenpunkt werden drei separate Experimente durchgeführt und der Mittelwert herangezogen. Die Ergebnisse hinsichtlich pHₘₐₓ und t_{G} sind in Figur 3 dargestellt. Das Phototrodensignal für ausgewählte Datenpunkte ist in Figur 4 dargestellt.

Ohne Zugabe von ortho-Phosphat setzt nach etwa zwei Stunden eine Ausfällung von Calciumcarbonat einsetzt, was an der Abnahme der Transmission der Figur 4 erkennbar ist. Die Zugabe von ortho-Phosphat hach Beginn der Fällung hat einen inhibierenden Effekt. Eine Auflösung des Niederschlags wird nicht beobachtet. Eine Zugabe nach Beginn der Fällung verlangsamt also den weiteren Ausfällungsprozess.

Der Figur 3 kann entnommen werden, dass es keine Wechselwirkung zwischen dem stabilisierenden Effekt und dem Zeitpunkt der Zugabe von ortho-Phosphat gibt. Die in Figur 3 erkennbare große Standardabweichung für den Messpunkt bei 105 Minuten resultiert daraus, dass eine maßgebliche Ausfällung bei zwei der drei Wiederholungen des Experiments bereits eingesetzt hatte.

### Beispiel 2:

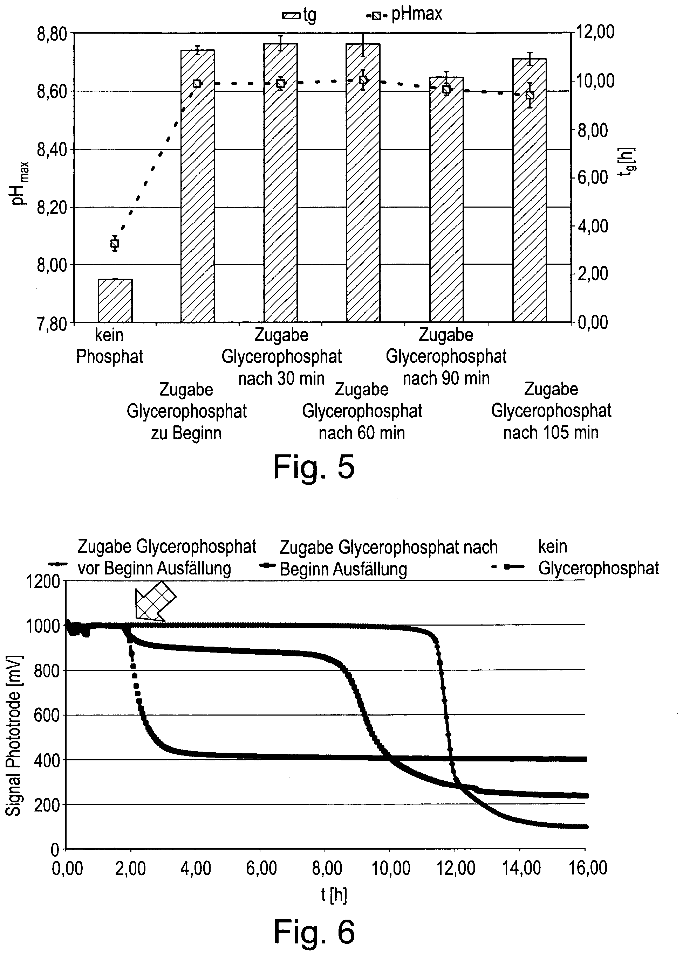

Das Experiment des Beispiels 1 wird in derselben Weise wiederholt, mit dem einzigen Unterschied, dass an Stelle von ortho-Phosphat diesmal Glycerophosphat zugegeben wird. Nämlich wird zu den Kolben enthaltend 250 ml DUOSOL 4551 nach einem bestimmten Zeitraum 2,5 ml einer Stammlösung mit 100 mmol/I Glycerophosphat zugesetzt, was im Kolben zu einer Konzentration von 1,0 mmol/I an Glycerophosphat führt. Die Ergebnisse hinsichtlich pHₘₐₓ und t_{G} sind in Figur 5 dargestellt. Das Phototrodensignal für ausgewählte Datenpunkte ist in Figur 6 dargestellt. Aus diesen Graphen lassen sich ähnliche Schlussfolgerungen ziehen, wie dies für Beispiel 1 anhand der Graphen der Figuren 3 und 4 gemacht wurde.

Zusammenfassend lässt sich festhalten, dass die zeitversetzte Zugabe von Phosphaten bzw. Phosphatestern zu calciumhaltigen und bicarbonatgepufferten Dialyselösungen denselben stabilisierenden Effekt gegenüber einer Ausfällung von Calciumcarbonat hat wie eine initiale Zugabe während der Lösungsherstellung. Selbst gealterte Lösungen mit erhöhtem pH-Wert können durch diese Vorgehensweise stabilisiert und somit sicherer gemacht werden. Dieses Prinzip funktioniert vor und auch während einer Dialysebehandlung.

Im Falle von organischen Phosphatestern und insbesondere Glycerophosphat ermöglicht die Zugabe von rund 1 mmol/I die Einstellung einer physiologischen Phosphatkonzentration unter gleichzeitiger Erhöhung der Stabilität gegenüber Ausfällungsreaktionen von Calciumcarbonaten.

## Patentansprüche

1. Verfahren zur Stabilisierung einer Dialyselösung, die Calciumionen sowie Bicarbonationen enthält,
**dadurch gekennzeichnet,**
**dass** der Dialyselösung in zeitlichem Abstand zu deren Herstellung ein Phosphat in einer Menge von bis zu 0,4 mmol/I und/oder ein organischer Phosphatester in einer Menge von bis zu 1,25 mmol/l zugegeben wird, der zeitliche Abstand zur Herstellung mehr als 30 Minuten, mehr als 60 Minuten, mehr als 90 Minuten oder mehr als 105 Minuten beträgt und der pH-Wert der Lösung im Zeitpunkt der nachträglichen Zugabe größer als 7,2 ist.

2. Verfahren nach Anspruch 1, **dadurch gekennzeichnet, dass** es sich bei dem Phosphat um ortho-Phosphat handelt und/oder dass es sich bei dem organischen Phosphatester um einen organischen Ester des ortho-Phosphats handelt.

3. Verfahren nach Anspruch 2, **dadurch gekennzeichnet, dass** es sich bei dem organischen Ester des ortho-Phosphats um ein Glycerin-ortho-Phosphat handelt.

4. Verfahren nach einem der vorhergehenden Ansprüche, **dadurch gekennzeichnet, dass** das Phosphat in einer Menge von mindestens 0,05 mmol/l zugegeben wird.

5. Verfahren nach einem der vorhergehenden Ansprüche, **dadurch gekennzeichnet, dass** der organische Phosphatester in einer Menge von mindestens 0,8 mmol/l zugegeben wird.

6. Verfahren nach einem der vorhergehenden Ansprüche, **dadurch gekennzeichnet, dass** der Lösung in zeitlichem Abstand zu deren Herstellung sowohl ein Phosphat als auch ein organischer Phosphatester zugegeben wird, wobei vorzugsweise vorgesehen ist, dass das Konzentrationsverhältnis zwischen dem Phosphat und dem organischen Phosphatester zwischen 0,3/0,7 und 0,9/0,1 liegt.

7. Verfahren nach einem der vorhergehenden Ansprüche, **dadurch gekennzeichnet, dass** der zeitliche Abstand zur Herstellung mehr als eine Woche, mehr als ein Monat, mehr als sechs Monate oder mehr als ein Jahr beträgt.

8. Verfahren nach einem der vorhergehenden Ansprüche, **dadurch gekennzeichnet, dass** der pH-Wert der Lösung im Zeitpunkt der nachträglichen Zugabe größer als 7,4 oder größer als 7,6 ist.

## Claims

1. A method of stabilizing a dialysis solution that includes calcium ions and bicarbonate ions,
**characterized in that**
a phosphate is added to the dialysis solution at a time interval from its preparation in an amount of up to 0.4 mmol/l and/or an organic phosphate ester is added to the dialysis solution at a time interval from its preparation in an amount of up to 1.25 mmol/l.; the time interval from the preparation amounts to more than 30 minutes, more than 60 minutes, more than 90 minutes, or more than 105 minutes; and the pH .of the solution at the time of the subsequent addition is greater than 7.2.

2. A method in accordance with claim 1, **characterized in that** the phosphate is an orthophosphate; and/or **in that** the organic phosphate ester is an organic ester of the orthophosphate.

3. A method in accordance with claim 2, **characterized in that** the organic ester of the orthophosphate is a glycerol orthophosphate.

4. A method in accordance with one of the preceding claims, **characterized in that** the phosphate is added in an amount of at least 0.05 mmol/l.

5. A method in accordance with one of the preceding claims, **characterized in that** the organic phosphate ester is added in an amount of at least 0.8 mmol/l.

6. A method in accordance with one of the preceding claims, **characterized in that** both a phosphate and an organic phosphate ester are added to the solution at a time interval from its preparation, with provision preferably being made that the concentration ratio between the phosphate and the organic phosphate ester is between 0.3/0.7 and 0.9/0.1.

7. A method in accordance with one of the preceding claims, **characterized in that** the time interval from the preparation amounts to more than one week, more than one month, more than six months, or more than one year.

8. A method in accordance with one of the preceding claims, **characterized in that** the pH of the solution at the time of the subsequent addition is greater than 7.4 or greater than 7.6

## Revendications

1. Procédé de stabilisation d'une solution de dialyse qui contient des ions calcium et des ions bicarbonate,
**caractérisé en ce que**
l'on ajoute à la solution de dialyse, à distance dans le temps de sa fabrication, un phosphate en une quantité de 0,4 mmol/I maximum et/ou un ester de phosphate organique en une quantité de 1,25 mmol/I maximum, la distance dans le temps par rapport à la fabrication est de plus de 30 minutes, plus de 60 minutes, plus de 90 minutes ou plus de 105 minutes et le pH de la solution au moment de l'ajout ultérieur est supérieur à 7,2.

2. Procédé selon la revendication 1, **caractérisé en ce que** le phosphate est un ortho-phosphate et/ou **en ce que** l'ester de phosphate organique est un ester organique de l'ortho-phosphate.

3. Procédé selon la revendication 2, **caractérisé en ce que** l'ester organique de l'ortho-phosphate est un glycérol-ortho-phosphate.

4. Procédé selon l'une des revendications précédentes, **caractérisé en ce que** le phosphate est ajouté en une quantité d'au moins 0,05 mmol/l.

5. Procédé selon l'une des revendications précédentes, **caractérisé en ce que** l'ester de phosphate organique est ajouté en une quantité d'au moins 0,8 mmol/I.

6. Procédé selon l'une des revendications précédentes, **caractérisée en ce que** l'on ajoute à la solution, à distance dans le temps de sa fabrication, un phosphate et un ester de phosphate organique, le rapport de concentration entre le phosphate et l'ester de phosphate organique étant de préférence prévu pour être compris dans l'intervalle de 0,3/0,7 à 0,9/0,1.

7. Procédé selon l'une des revendications précédentes, **caractérisé en ce que** la distance dans le temps par rapport à la fabrication est de plus d'une semaine, plus d'un mois, plus de six mois ou plus d'un an.

8. Procédé selon l'une des revendications précédentes, **caractérisé en ce que** le pH de la solution au moment de l'ajout ultérieur est supérieur à 7,4 ou supérieur à 7,6.
